(19) Europäisches Patentamt
European Patent Office
Office européen des brevets

(11) Publication number: 0 237 233 A2

(12) EUROPEAN PATENT APPLICATION

(21) Application number: 87301656.2

(51) Int. Cl.4: A61K 6/08

(22) Date of filing: 25.02.87

(30) Priority: 28.02.86 US 835035

(43) Date of publication of application: 16.09.87 Bulletin 87/38

(84) Designated Contracting States: CH DE ES FR GB IT LI NL SE

(71) Applicant: MINNESOTA MINING AND MANUFACTURING COMPANY
3M Center, P.O. Box 33427
St. Paul, Minnesota 55133-3427(US)

(72) Inventor: Oxman, Joel D. c/o Minnesota Mining and
Manufacturing Co. 2501 Hudson Road P.O. Box 33427
St. Paul Minnesota 55133-3427(US)
Inventor: Ubel, Frank A. III
Manufacturing Co. 2501 Hudson Road P.O. Box 33427
St. Paul Minnesota 55133-3427(US)

(74) Representative: Baillie, Iain Cameron et al
c/o Ladas & Parry Isartorplatz 5
D-8000 München 2(DE)

# (54) One-part visible light-curable dentin enamel adhesive.

(57) Light-curable dental adhesive compositions containing a phosphorus-containing free radically polymerizable compound, chromophore-substituted halomethyl-s-triazine or oxadiazole, and an optional ketone. The compositions adhere well to dental substrates (e.g., dentin) and have excellent shelf life when formulated as a one-part composition.

EP 0 237 233 A2

Xerox Copy Centre

# ONE-PART VISIBLE LIGHT-CURABLE DENTIN AND ENAMEL ADHESIVE

## Technical Field

This invention relates to light-curable compositions useful in dentistry.

## Background Art

Practitioners in the field of dentistry have long sought polymerizable compositions that would adhere well to dentin. A number of compositions that employ phosphorus-containing free-radically polymerizable compounds have been reported in the literature, see, e.g, M. Buonocore, W. Wileman, and F. Brudevold, J. Dent. Res., 35, 846 (1956), M. Buonocore and M. Quigley, J. Amer. Dent. Assoc., 57, 807 (1958), M. Anbar and E. Farley, J. Dent. Res., 53, 879 (1974), E. Farley, R. Jones, and M. Anbar, J. Dent. Res., 56, 1943 - (1977), U.S. Pat. Nos. 3,882,600, 3,997,504, 4,222,780, 4,235,633, 4,259,075, 4,259,117, 4,368,043, 4,383,052, 4,499,251, 4,514,342, 4,515,930, 4,537,940, 4,539,382, and 4,544,467, European published patent application No. 0 058 483, and Japanese laid-open patent application (Kokai) Nos. 57-143372 and 57-167364. These compositions typically are sold in the form of autocure or light-cure compositions. The light-cure compositions typically contain camphorquinone as a photoinitiator. Most of the light-cure compositions are sold in a two-part configuration (exceptions being the one-part products "Sinterbond" from Teledyne Getz, and "Caulk Universal Bond" from Dentsply, Intl., Inc.), since two-part packaging typically provides optimum bonding performance and maximum shelf life.

Certain halogenated triazines said to be useful as dentin and enamel adhesives are described in U.S. Pat. No. 4,203,220. The triazines of that patent are not photoinitiators.

## Summary of Invention

In practice, it has proved very difficult to formulate many of the above-described phosphorus compounds in a one-part visible light-curable dental adhesive composition. When the phosphorus compound is acidic, one or more components of the photoinitiator system tend to decompose, become protonated, and/or experience a shift in the desired spectral response. In addition, several photoinitiation systems that provide good bonding performance in two-part systems have poor shelf life when formulated as a one-part composition.

The present invention provides a light-curable dental adhesive composition comprising phosphorus-containing free-radically polymerizable compound suitable for use in the oral environment, photoinitiator, and optional ketone, characterized in that said photoinitiator comprises chromophore-substituted halomethyl-s-triazine or chromophore-substituted halomethyl-oxadiazole. The compositions of the invention exhibit excellent dentin bonding performance, and excellent shelf life when formulated as a one-part composition. Preferred compositions of the invention cure upon exposure to visible light (wavelengths of about 400 to about 760 nanometers) or near-UV light (wavelengths of about 330 to about 400 nanometers) to provide a hard film that is reasonably insensitive to the presence of oxygen during cure and enables formation of strong bonds to dentin, enamel, cementum, dental metals and alloys, porcelain, glass, and dental composites, restoratives, and adhesives.

## Detailed Description

In the practice of the present invention, the phosphorus-containing free-radically polymerizable compound (hereafter sometimes referred to as the "phosphorus compound") is a monomer, oligomer, or polymer (or mixture thereof) which is suitable for use in the oral environment, both in its unpolymerized and polymerized state. Preferably the phosphorus compound is a liquid having sufficiently low viscosity to enable it, when used alone or with an optional diluent, to be applied to exposed tooth surfaces using, for example, a small brush, dropper or syringe. Using the shear strength test recited in Example 1 of European published patent application No. 0 058 543, the phosphorus compound preferably has an initial average shear strength on unetched dentin of at least 5 kg/cm$^2$, more preferably at least 20 kg/cm$^2$.

Preferred phosphorus compounds for use in the present invention contain one or more phosphorus atoms bonded, through a P-OR or P-R linkage where R is a carbon, nitrogen or sulfur atom, to a hydrocarbyl radical containing one or more olefinically unsaturated groups. Preferred olefinically unsaturated groups are methacryl and acryl radicals. Preferably, one or more phosphorus atoms of the phosphorus compound also are bonded to one or more (a) halogen atoms through a P-Cl, P-Br or P-F linkage, (b) active hydrogen atoms through a P-H or P-OH linkage, or (c) substituted or unsubstituted hydrocarbyl groups - (e.g., an alkyl, aryl, alkaryl, or aryalkyl group) through a P-OR or P-R linkage where R is as defined above. A particularly preferred class of phosphorus compound is described in European Patent No. 0 058 483 and U.S. Pat. No. 4,515,930. The phosphorus compounds of this class are halophosphorus acid esters of chlorine-containing or bromine-containing phosphorus acids. An especially preferred subclass of such halophosphorus acid esters contains halophosphorus acid esters of diglycidyl methacrylate of Bisphenol A ("Bis-GMA") prepared by reaction of Bis-GMA with a chlorine-containing or bromine-containing phosphorus acid. Especially preferred phosphorus acids which can be reacted with Bis-GMA include $POCl_3$, $PCl_3$, $R'_{1\ or\ 2}OP(O)Cl_{2\ or\ 1}$ (where R′ is a hydrocarbyl radical, preferably one derived by the removal of one or more hydroxyl groups from a hydroxyl-containing compound such as 2-hydroxyethyl methacrylate, ethylene glycol, polyethylene glycol, pentaerythritol, and the like), and $PBr_3$.

An additional preferred class of phosphorus compounds for use in the present invention contains the phosphorus acid esters described in U.S. Pat. Nos. 3,882,600, 3,997,504, 4,222,780, 4,235,633, 4,259,075, 4,259,117, 4,368,043, 4,442,239, 4,499,251, 4,514,342, 4,537,940, 4,539,382 and Japanese published patent application (Koho) No. 85-17235. Especially preferred members of such class are the compounds
2-methacryloyloxyethyl phenyl phosphate and
10-methacryloyloxydecyl dihydrogen phosphate.

A further preferred class of phosphorus compounds for use in the present invention contains the pyrophosphate ester derivatives described in U.S. Pat. Nos. 4,383,052 and 4,404,150 and in Japanese laid-open application Nos. 57-143372 and 57-167364.

A further preferred phosphorus compound is glycerophosphate dimethacrylate as described in the above-mentioned Buonocore, Wileman, and Brudevold publication.

Either a single phosphorus compound or a mixture of phosphorus compounds can be used in this invention. If desired, other free-radically polymerizable non-phosphorus-containing compounds can be mixed therewith, for example, as a diluent to reduce viscosity or promote wetting. Other suitable free-radically polymerizable compounds include mono-or poly-(e.g., di-, tri-or tetra-functional) acrylates and methacrylates such as methyl acrylate, 2-hydroxyethyl acrylate, triethyleneglycol diacrylate, neopentylglycol diacrylate, hexamethyleneglycol diacrylate, trimethylolpropane triacrylate, pentaerythritol tetraacrylate, polyalkylene glycol mono-and di-acrylates, urethane mono-or poly-functional acrylates, Bisphenol A diacrylates, and the corresponding methacrylates of the above compounds, as well as acrylamides and methacrylamides, vinyl compounds, styrene compounds, and other olefinically unsaturated compounds suitable for use in the oral environment. U.S. Pat. Nos. 4,499,251, 4,515,930, 4,537,940 and 4,539,382 contain an extensive list of such compounds.

The phosphorus compound can be prepared using methods known to those skilled in the art. It can also be obtained from existing commercially available dental adhesives. Suitable commercially available dental adhesives include "Scotchbond" dental adhesive and "Light Cured Scotchbond" dental adhesive (both commercially available from 3M), "Bondlite" dental adhesive (commercially available from Sybron Corp.), "Caulk Universal Bond" dental adhesive (commercially available from Dentsply Intl., Inc.), "Clearil F" dental adhesive and "Clearfil New Bond" dental adhesive (both commercially available from Kuraray Co., Ltd.), "Johnson & Johnson" dentin bonding agent and "Johnson & Johnson" light-curing bonding agent (both commercially available from Johnson & Johnson Co.), "Palfique" bonding agent (commercially available from Tokuyama Soda Co., Ltd.), "Shofu" bonding base (commercially available from Shofu, Inc.), and "Sinterbond" dental adhesive (commercially available from Teledyne Getz).

Preferably, the phosphorus compound constitutes about 1 to about 99 percent of the weight of compositions of the invention, more preferably about 25 to about 95 weight percent. In general, the weight of other components of the compositions of the invention can be determined by reference to the total weight of phosphorus compound and other free-radically polymerizable compounds therein.

The chromophore-substituted halomethyl-s-triazine (hereafter sometimes referred to as the "triazine") or chromophore-substituted halomethyl-oxadiazole (hereafter sometimes referred to as the "oxadiazole") absorbs visible or ultraviolet light and initiates free radical polymerization. The chromophoric moiety renders the triazine or oxadiazole capable of absorbing light of the desired wavelength or wavelengths. The triazine contains at least one halomethyl group and at least one chromophoric moiety. The oxadiazole contains one each of the halomethyl group and chromophoric moiety.

The triazine or oxadiazole (hereafter sometimes referred to collectively as the "photoinitiator") can be selected by measuring its absorption spectra (using, e.g., a laboratory spectrophotometer) and calculating its extinction coefficient according to Beer's law at the desired wavelength or wavelengths of irradiation. Preferably, the photoinitiator absorbs sufficient light having a wavelength between about 400 to about 500 nanometers to enable a composition of the invention to undergo free radical polymerization using a standard dental visible light curing device and an irradiation time of less than one minute. If the photoinitiator has insufficient absorption within this range, then preferably it is used together with the aforementioned optional ketone. Use of a photoinitiator having a low extinction coefficient at the desired wavelength or wavelengths of irradiation will facilitate the curing of thick films.

The halomethyl group preferably is a trihalomethyl group. Most preferably, the halomethyl group is a trichloromethyl group.

Alteration of the chromophoric moiety affects not only the spectral absorption and extinction coefficient of the photoinitiator but also its solubility in the phosphorus compound, and thus the chromophoric moiety should be selected on the basis of absorption, extinction coefficient and solubility. For applications where the compositions of the invention will be discernible after cure (e.g., for sealants, repair of shallow caries, and root desensitization of anterior teeth), then the color of any residual photoinitiator or decomposition products thereof should also be taken into account, and thus the chromophoric moiety should be selected with a view to the aesthetic appearance of the cured composition.

The chromophoric moiety preferably is a substituted or unsubstituted styryl, aryl (e.g., phenyl), or alkyl (e.g., methyl, ethyl, or halomethyl) group. Especially preferred chromophoric moieties render the photoinitiator capable of absorbing visible light. Alkyl or alkoxy-substituted styryl groups are particularly preferred chromophoric moieties.

A preferred subclass of triazines has the formula:

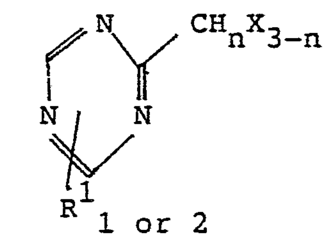

I

where n is zero to two, each X is independently chlorine or bromine, and each R' is independently a chromophore of the type described above. Preferably in triazines of Formula I above, n is zero, X is chlorine, and R' is substituted or unsubstituted styryl. Most preferably, R' is alkyl-or alkoxy-(e.g., methoxy-) substituted styryl.

Preferred triazines are also described in U.S. Pat. Nos. 3,954,475, 3,987,037, 4,189,323, 4,239,850, 4,258,123, 4,259,432, 4,329,384, 4,330,590, 4,391,687, 4,399,211, 4,476,215 and 4,481,276.

Representative triazines which can be used in the present invention include 2-styryl-4,6-bis(trichloromethyl)-s-triazine, 2-(p-chlorostyryl)-4,6-bis(trichloromethyl)-s-triazine, 2-(p-methylstyryl)-4,6-bis(trichloromethyl)-s-triazine, 2-(o -methylstyryl)-4,6-bis(trichloromethyl)-s-triazine, 2-(2,4-dimethylstyryl)-4,6-bis(trichloromethyl)-s-triazine, 2-(p-methoxystyryl)-4,6-bis (trichloromethyl)-s-triazine, 2-(m-methoxystyryl)-4,6-bis(trichloromethyl)-s-triazine, 2-phenyl-4,6-bis(chloromethyl)-s-triazine, 2-(p-chlorophenyl)-4,6-bis(dibromomethyl)-s-triazine, 2-(p-tolyl)-4,6-bis(trichloromethyl)-s-triazine, 2-(p -methoxyphenyl)-4,6-bis(trichloromethyl)-s-triazine, 2-(2',4'-dichlorophenyl)-4,6-bis(trichloromethyl)-s-triazine, 2-(4-methoxy-1-naphthylvinylene)-4,6-bis(trichloromethyl)-s-triazine,
2-(naphth-1-yl)-4,6-bis(trichloromethyl)-s-triazine, 2-(4-methoxy-naphth-1-yl)-4,6-bis(trichloromethyl)-s-triazine, 2-(4-(2-ethoxy-ethoxy)-naphth 1-yl))-4,6-bis(trichloromethyl)-s-triazine, 2-(1-methoxy-naphth-2-yl)-4,6-bis(trichloromethyl)-s-triazine, 2-(6-methoxy-naphth-2-yl)-4,6-bis(trichloromethyl)-s-triazine, 2,4,6-tris-(trichloromethyl)-s-triazine, 2-methyl-4,6-bis (trichloromethyl)-s-triazine, 2-n-nonyl-4,6-bis(trichloromethyl)-s-triazine,
2-($\alpha$,$\alpha$,$\beta$-trichloroethyl)-4,6-bis(trichloromethyl)-s-triazin e, 2-methyl-4,6-bis(tribromomethyl)-s-triazine, 2,4,6-tris (tribromomethyl)-s -triazine,
2-methyl-4-amino-6-tribromomethyl-s-triazine, 2-methyl-4-methoxy-6-trichloromethyl-s-triazine, and mixtures thereof.

The triazines can be prepared using methods known to those skilled in the art. Such methods are described in detail in U.S. Pat. Nos. 3,954,475, 3,987,077, 4,189,323, and 4,239,850.

A preferred subclass of oxadiazoles has the formula:

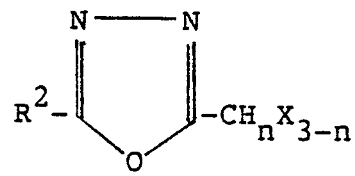

II

where n and X are as defined above, and $R^2$ is a chromophore of the type described above. Preferably in oxadiazoles of Formula II above, n is zero, X is chlorine, and $R^2$ is substituted or unsubstituted styryl. Most preferably, $R^2$ is alkyl-or alkoxy-substituted styryl.

Preferred oxadiazoles are also described in U.S. Pat. Nos. 4,212,970, 4,232,106, and 4,279,982.

Representative oxadiazoles which can be used in the present invention include 2-trichloromethyl-5-(p-methoxystryl)-1,3,4-oxadiazole and 2-trichloromethyl-5-(3′,4′-dimethoxystyryl)-1,3,4-oxadiazole.

The oxadiazoles can be prepared using methods known to those skilled in the art. Such methods are described in detail in U.S. Pat. Nos. 4,212,970, 4,232,106, and 4,279,982.

Mixtures of triazines and oxadiazoles can be used if desired. Triazines and oxadiazoles which cure on exposure to visible light are particularly preferred. The compounds 2-(p-methoxystyryl)-4,6-bis-(trichloromethyl)-s-triazine, 2-(p-methylstyryl)-4,6-bis(trichloromethyl)-s-triazine, 2-(2,4-dimethylstyryl)-4,6-bis(trichloromethyl)-s-triazine, 2-(m-methoxystyryl)-4,6-bis(trichloromethyl)-s-triazine, and 2-trichloromethyl-5-(3′,4′-dimethoxystyryl)-1,3,4-oxadiazole are especially preferred photoinitiators for use in this invention.

The composition of the invention contains an "effective amount" of photoinitiator, that is, sufficient photoinitiator to enable cure of the composition when it is exposed to light of a desired wavelength and intensity for a desired period of time. Most preferably, light from a visible light curing unit of the type commonly used in dentistry is employed, with exposure times of 1 minute or less, more preferably 20 seconds or less. Preferred commercially available curing units include the "Visilux" and "Visilux 2" curing lights (both commercially available from 3M), the "Optilux" curing light (commercially available from Demetron Research Corporation), and the "Elipar" and "ESPE Visio-Beta" curing lights (both commercially available from Premier Dental Products Company). The photoinitiator can be present in compositions of the invention in amounts as high as the limit of solubility but lower amounts preferably are used. The amount of photoinitiator preferably is adjusted according to the spectral output and intensity of the curing unit employed. When a large amount of photoinitiator is employed, absorption is increased and inadequate penetration of photolytic energy through a film of the composition may result, with a concomitant decrease in adhesive bond strength. The amount of photoinitiator which should be used will depend primarily upon factors such as the other ingredients present in the composition, the wavelength and intensity of irradiation, and the film thickness of the composition, and thus only a general guide to the photoinitiator amount can be given. Based on the total weight of phosphorus compound and other free-radically polymerizable compounds present in the composition, a preferred amount of photoinitiator is about 0.05 to about 5 weight percent, more preferably about 0.1 to about 1 weight percent.

It is optional (but preferred) to include a ketone in compositions of the invention, preferably a monoketone or diketone. The ketone enhances the bonding strength of the composition on dentin, thus acting as a synergist. For photoinitiators that do not by themselves absorb visible light, the ketone can facilitate the decomposition of the photoinitiator to form free radicals in the presence of visible light, thus acting as a spectral sensitizer. Accordingly, the ketone enhances bonding strength and/or absorbs light in the range of about 400 to 700 nanometers. Suitable monoketones include substituted or unsubstituted alkylphenones (e.g., acetophenone), benzylidene acetophenones (e.g., chalcone), benzophenones, benzoins, fluorenones, anthrones, benzanthrones, thioxanthones, thioxanthone oxides, thioxanthone dioxides, xanthones, acridones, and aminoketones (e.g., 4-(dimethylamino)benzophenone). Suitable diketones include substituted or unsubstituted biacetyls, benzils, naphthaquinones, bornanediones (e.g., camphorquinone), acenaphthenequinones, phenanthrenequinones, furils, and anthraquinones. Mixtures of ketones can be used if desired. Representative ketones are described in U.S. Pat. Nos. 3,427,161, 3,756,827, 3,759,807, 4,071,424, 4,544,467, and Re 28,789. Alpha-diketones are preferred. Especially preferred α-diketones are camphorquinone, acenaphthenequinone, and phenanthrenequinone. An additional preferred ketone is 1,4-naphthaquinone.

When an optional ketone is employed in a composition of the invention, it preferably is present in an amount sufficient to enhance the bond strength of the composition on dentin when such composition is exposed to visible light of a desired wavelength and intensity for a desired period of time. The ketone can be present in compositions of the invention in amounts as high as the limit of solubility, but lower amounts preferably are used. The amount of ketone preferably is adjusted according to the type and amount of photoinitiator and the spectral output and intensity of the curing unit employed. The amount of ketone that

should be used will depend primarily upon factors such as the other ingredients present in the composition, the wavelength and intensity of irradiation, and the film thickness of the composition, and thus only a general guide to the ketone amount can be provided. Expressed on a weight basis, a preferred amount of ketone is about 0.05 to 5 percent, more preferably 0.1 to 1 percent, of the weight of the composition of the invention.

If desired, one or more peroxide compounds can also be added to compositions of the invention, in order to enhance bonding performance. Suitable peroxide compounds include acyl and alkyl peroxides and hydroperoxides and diacyl and dialkyl peroxides, e.g., benzoyl peroxide, t-butyl hydroperoxide, acetyl peroxide, lauroyl peroxide, and the like.

If desired, one or more sulfur compounds having sulfur in the $^{+2}$ or the $^{+4}$ oxidation state can also be added to compositions of the invention, in order to enhance bonding performance. As used herein, the term "oxidation state" is as defined in Hendrickson et al., Organic Chemistry, 3d Ed., 798-799 (1970). Suitable sulfur compounds include alkali metal salts (e.g., potassium or sodium salts) or ammonium salts of sulfur-containing anions such as sulfinate, sulfite, bisulfite, metabisulfite, or hydrosulfite anions or the free acid counterparts thereof. Such sulfur compounds include sodium benzene sulfinate, sodium p-toluene sulfinate, tetrabutylammonium bisulfite, potassium metabisulfite, p-toluene sulfinic acid, and the like, and mixtures thereof. Preferably the sulfur compound is not used together with a peroxide compound if the composition of the invention is intended to be stored before use.

Other adjuvants such as diluents, solvents, fluorides (e.g., sodium fluoride, potassium fluoride, magnesium fluoride, calcium fluoride, aluminum fluoride, or zirconium fluoride), wetting agents, fillers (e.g., finely ground quartz, fumed or colloidal silica, or non-vitreous microparticles of the type described in U.S. Pat. No. 4,503,169, preferably silane-treated to ensure coupling of the filler to the cured composition of the invention), pigments, indicators, inhibitors, stabilizers, UV absorbers and the like can also be added to compositions of the invention. The amounts and types of such adjuvants, and their manner of addition to the compositions of the invention, will be essentially the same as currently used in existing dental materials familiar to those skilled in the art.

Compositions of the invention preferably are put up in vials, syringes or compules. The compositions can be applied to tooth tissue, dental prosthetic devices, existing (cured) dental composites and restoratives, orthodontic brackets, dental metals and alloys, glass, and other substrates. When used on teeth, the compositions of the invention are applied to dentin, enamel, or cementum in a manner similar to that used for existing dental adhesives. Excavation can be limited to the removal of damaged or defective tooth tissue. Undercutting cavities generally is not required. Preferably, enamel margins (if present) are acid-etched using conventional etchants, but acid etching of dentin or cementum is avoided.

For tooth veneering or restoration, one or more thin layers of a dental adhesive composition of the invention preferably are applied to freshly exposed, water-rinsed, air-dried tooth tissue and exposed (layer by layer or all at once) to a sufficient quantity and intensity of visible light to cure the dental adhesive to a hard film. Next, enamel margins (if present) are beveled using a suitable abrasive device and etched using a conventional etchant. The etchant is removed with a water rinse and the etched enamel margin is air dried. One or more additional thin layers of the dental adhesive are applied to the area of the restoration and cured using visible light. Optionally, an autocured or visible light-cured dental composite, dental restorative, or orthodontic adhesive composition is applied to the hardened dental adhesive, optionally followed by the application of a dental prosthetic device (e.g., an inlay, onlay or crown) or tooth repositioning device (e.g., an orthodontic bracket).

If desired, pretreatments (see, e.g., U.S. Pat. Nos. 4,251,565 and 4,538,990) can be used on dentin and enamel. Pretreatments which have been tried include water, ethanol, acetone, methylene chloride, ethylenediaminetetraacetic acid (EDTA), sodium hypochlorite, sodium phosphate, sodium oxalate, potassium iodate, citric acid, phosphoric acid, hydrogen peroxide, and 2-hydroxyethylmethacrylate.

Compositions of the invention can also be applied to previously-cured dental composites or restoratives for the repair or augmentation thereof. In addition, compositions of the invention can be applied to porcelain, dental metals and alloys, and glass and used, e.g., for the repair of broken crowns.

The following examples are offered to aid understanding of the present invention and are not to be construed as limiting the scope thereof. Unless otherwise indicated, all parts and percentages are by weight.

## EXAMPLE 1

Adhesion to Dentin

A refrigerated resin mixture containing 95 parts Bis-GMA and its isomers, 95 parts triethyleneglycol dimethacrylate, 2 parts benzoyl peroxide and 0.13 part butylated hydroxytoluene was stirred in a flask with 10 parts phosphorus oxychloride for 3 hours while keeping the temperature of the reaction mixture below 24°C. The reaction mixture was allowed to warm to room temperature and stand for 8 days, at which time its viscosity was measured and found to be approximately 3 Pa•s at 20°C. The product was a clear, syrupy, phosphorus compound-containing adduct. A portion of this adduct was set aside as a control.

Three one-part visible light curable dental adhesives were prepared by adding 0.5 percent each of camphorquinone ("CPQ"), 2-p -methoxystyryl-4,6-bis(trichloromethyl)-s-triazine ("MOST"), or CPQ and MOST to portions of the above described adduct. The resulting mixtures were stirred to dissolve the added ingredients and stored in polyethylene bottles. Adhesion to unetched dentin was evaluated using the following procedure. Five bovine teeth of similar age and appearance were partially embedded in circular acrylic discs. The exposed portion of each tooth was ground flat and parallel to the acrylic disc using Grade 120 silicon carbide paper-backed abrasive mounted on a lapidary wheel, in order to expose the dentin. During this and subsequent grinding and polishing steps, the teeth were continuously rinsed with water. Further grinding and polishing of the teeth was carried out by mounting Grade 320 silicon carbide paper-backed abrasive and then Grade 600 silicon carbide paper-backed abrasive on the lapidary wheel. The polished teeth were stored in distilled water, and used for testing within 2 hours after polishing. The polished teeth were removed from the water and dried using a stream of compressed air. A single drop of one of the above-described dental adhesives was painted onto each of the polished tooth surfaces with a brush and blown lightly into a film with compressed air. The adhesive was cured using a 10-second irradiation with a "Visilux" dental curing light. Previously prepared molds made from a 2-mm thick "Teflon" sheet with a 5-mm diameter hole through the sheet were clamped to each polished tooth so that the central axis of the hole in the mold was normal to the polished, dental adhesive-coated tooth surface. The hole in each mold was filled with a visible light-cureable dental restorative ("Silux" brand restorative, universal shade, commercially available from 3M) and cured using a 30-second irradiation. The teeth and molds were allowed to stand for about 5 minutes at room temperature, then stored in distilled water at 37°C for 24 hours. The molds were then carefully removed from the teeth, leaving a molded button of restorative attached to each tooth.

Adhesive strength was evaluated by mounting the acrylic disk in a holder clamped in the jaws of an "Instron" apparatus, with the polished tooth surface oriented parallel to the direction of pull. A loop of orthodontic wire (0.44 mm diameter) was placed around the restorative button adjacent to the polished tooth surface. The ends of the orthodontic wire were clamped in the pulling jaw of the Instron apparatus, thereby placing the bond in shear stress. The bond was stressed until it failed, using a crosshead speed of 2 mm/min. Set out below in TABLE I are the run number, ingredients and average measured shear strength for the control composition and the three dental adhesives.

TABLE I

| Run | Ingredients | Adhesion, $kg/cm^2$ |
|---|---|---|
| 1 | Adduct alone (control) | 0 |
| 2 | Adduct plus 0.5% CPQ | 23.8 |
| 3 | Adduct plus 0.5% MOST | 61.1 |
| 4 | Adduct plus 0.5% each of MOST and CPQ | 84.8 |

For the purpose of a further comparison, a two-part visible light-cure dental adhesive was formulated and similarly evaluated. The first part of the dental adhesive contained the adduct described above. The second part contained 3% sodium benzene sulfinate and 0.15% camphorquinone in ethanol. The two parts were mixed, then applied to dentin and cured as described above. The cured adhesive film was soft and had a thick inhibition layer, indicating that a relatively low degree of polymerization had been obtained. Hard

films with very thin inhibition layers were obtained for the compositions of the invention (Runs 3 and 4 above), indicative of reduced air inhibition and/or a higher degree of polymerization. The cured film of two-part adhesive was covered with restorative and evaluated as described above. The measured adhesive shear strength on dentin (average of over 50 teeth) was 47.9 kg/cm$^2$.

The above data demonstrates the improvement in adhesion to dentin obtained by employing a triazine photoinitiator. Combination of the triazine with the $\alpha$-diketone camphorquinone gave an especially desirable improvement in adhesion.

## EXAMPLE 2

Adhesion to Enamel

Using bovine enamel in place of dentin in the procedure of EXAMPLE 1, the average measured adhesive shear strength of the composition of Run 3 of EXAMPLE 1 on unetched enamel was 63.4 kg/cm$^2$. The average measured adhesive shear strength of this composition on enamel etched for 60 seconds with 35% $\sigma$-phosphoric acid was 121.6 kg/cm$^2$.

## EXAMPLE 3

Hydrolytic Stability

Bonded assemblies prepared as described in Run 3 of EXAMPLE 1 were stored in distilled water at 37°C for various periods of time and evaluated for adhesion to dentin. After one day, one week, one month and four months, the respective average measured adhesive shear strengths were 56.7, 94.9, 92.5 and 87.0 kg/cm$^2$. This example illustrates that the dental adhesive of the invention provides high initial bond strength that is maintained after water immersion.

## EXAMPLE 4

Storage Stability

The dental adhesive of Run 3 of EXAMPLE 1 was stored at room temperature (24°C) and at elevated temperature (45°C) for various periods of time. No visible increase in viscosity occured during storage. The dental adhesive was periodically removed from storage and evaluated for adhesion to dentin as described in EXAMPLE 1. Set out below in TABLE II are the storage temperature, storage time, and average measured adhesive shear strength on unetched dentin for each observation.

TABLE II

| Storage temperature | Storage time | Adhesion, kg/cm$^2$ |
|---|---|---|
| 24°C | 2 weeks | 61.6 |
| 24°C | 4 weeks | 56.0 |
| 45°C | 2 weeks | 78.0 |
| 45°C | 4 weeks | 70.7 |

The above data illustrates that the dental adhesive of the invention has good thermal stability and shelf life.

**EXAMPLE 5**

The procedure of Run 3 of EXAMPLE 1 was repeated using varying amounts of photoinitiator. The resulting compositions were evaluated for adhesion to dentin using a variety of cure procedures and five to ten tooth samples per evaluation. The cure procedures employed one or two layers of dental adhesive, with the first layer optionally being cured by irradiation with visible light for varying periods of time before application of additional dental adhesive or restorative. A button of dental restorative was then applied to the layer or layers of dental adhesive using the procedure of EXAMPLE 1, then finally cured by irradiation with visible light for varying periods of time. Set out below in TABLE III are the cure procedures employed in this example or in the following examples, the number of layers of dental adhesive, the cure time for the first layer of dental adhesive, and the final cure time.

TABLE III

| Cure procedure | No. of adhesive layers | Cure time for first adhesive layer, sec. | Final cure time, sec. |
|---|---|---|---|
| Method I | 2 | 20 | 20 |
| Method II | 2 | 30 | 20 |
| Method III | 2 | 20 | 30 |
| Method IV | 1 | 0 | 30 |
| Method V | 1 | 10 | 30 |
| Method VI | 1 | 20 | 20 |
| Method VII | 1 | 30 | 20 |
| Method VIII | 1 | 60 | 20 |

Set out below in TABLE IV are the run number, percent photoinitiator, and the average measured adhesive shear strength obtained by using Methods I through V.

TABLE IV

| | | Adhesion, kg/cm$^2$ | | | | |
|---|---|---|---|---|---|---|
| Run | % Photo-initiator | Method I | Method II | Method III | Method IV | Method V |
| 1 | 0.25 | 93.3 | 73.6 | 64.4 | - | - |
| 2 | 0.50 | 67.8 | 70.3 | 54.3 | 37.5 | 66.1 |
| 3 | 0.60 | - | - | - | 32.3 | 57.0 |
| 4 | 0.70 | - | - | - | 43.5 | 72.3 |
| 5 | 0.75 | 67.7 | 63.8 | 73.0 | - | - |
| 6 | 1.00 | 70.5 | 54.2 | 65.4 | - | - |

The above data shows the effect of variations in the amount of photoinitiator and the method of cure.

**EXAMPLE 6**

Using Method VI (unless otherwise noted below), adhesion to dentin was measured for compositions containing a variety of photoinitiators, and optionally containing 0.5% of various ketones. Set out below in TABLE V are the run number, amount and type of photoinitiator, identity of the ketone (if present), and the average measured adhesive shear strength on unetched dentin for each composition.

TABLE V

| Run | Amount and type of photoinitiator | Ketone | Adhesion, $kg/cm^2$ |
|---|---|---|---|
| 1 | 0.5% MOST | -- | 58.7 |
| 2 | 0.5% MOST | CPQ | 78.8 |
| 3 | 0.5% MOST | CPQ | 80.0(1) |
| 4 | 3% MOST | PAQ(2) | 62.4(1) |
| 5 | 0.5% 2-(m-methoxystyryl)-4,6-bis-(trichloromethyl)-s-triazine | -- | 57.4 |
| 6 | 0.5% MST(3) | -- | 58.6 |
| 7 | 0.5% MST | CPQ | 81.2 |
| 8 | 0.5% 2-(2,4-dimethylstyryl)-4,6-bis-(trichloromethyl)-s-triazine | -- | 51.9 |
| 9 | 0.5% 2-trichloromethyl-5-(3′,4′-dimethoxystyryl)-1,3,4-oxadiazole | -- | 70.2 |
| 10 | 0.5% MOSO(4) | -- | 8.6 |
| 11 | 0.5% MOSO | CPQ | 81.8 |

Notes for entries in TABLE V:

(1) Cured using Method VII.

(2) Phenanthrenequinone.

(3) 2-(p-methylstyryl)-4,6-bis(trichloromethyl)-s-triazine.

(4) 2-trichloromethyl-5-(p-methoxystyryl)-1,3,4-oxadiazole.

The above example illustrates the use of a variety of photoinitiators, and the improvement in adhesion obtained by combining the photoinitiator with a ketone. It is believed that the adhesion value of 8.6 kg/cm$^2$ obtained for Run 10 would have been higher if a near-UV light source had been used to effect cure.

**EXAMPLE 7**

Several commercially available phosphorus-containing two-part dental adhesive products were modified by adding photoinitiator to the resinous portion of each product. Set out below in TABLE VI are a letter identifying each product used, together with the product name, manufacturer, and the type of cure system.

TABLE VI

| Identifier | Product | Manufacturer | Cure type |
|---|---|---|---|
| A | "Scotchbond" dental adhesive | 3M | autocure |
| B | "Bondlite" | Kerr Division of Sybron Corp. | light cure |
| C | "Clearfil New Bond" dental adhesive | Kuraray Co., Ltd. | autocure |
| D | "Johnson & Johnson" dentin bonding agent | Johnson & Johnson Co. | autocure |
| E | "Johnson & Johnson" light-curing bonding agent | Johnson & Johnson Co. | light cure |
| F | "Palfique" bonding agent | Tokuyama Soda Co., Ltd. | autocure |
| G | "Shofu" bonding base | Shofu, Inc. | autocure |

The resinous ("Universal") portion of each product was modified by addition of 0.5 percent MOST. Without using the liquid ("Catalyst") portion of each product, the modified dental adhesives were evaluated for adhesion to dentin using Method VI. Adhesion to glass was also evaluated, by applying a layer of the modified adhesive to a clean glass microscope slide, placing a clean 4.0 mm diameter $\times$ 6.0 mm high glass button on the adhesive layer, and curing the resulting assembly for 20 seconds.

Set out below in TABLE VII are the product identifier, adhesion of the unmodified dental adhesive to dentin and glass (for this evaluation, both portions of each product were used, and the manufacturer's instructions for application and curing were followed), and adhesion of the modified dental adhesive to dentin and glass.

TABLE VII

| | Adhesion, kg/cm$^2$ | | | |
|---|---|---|---|---|
| | Unmodified adhesive | | Modified adhesive | |
| Product identifier | Dentin | Glass | Dentin | Glass |
| A | 44.4 | 90.4 | 68.0 | 110.2 |
| B | 55.6 | 103.7 | 37.1 | 80.1 |
| C | 23.8 | 42.5 | 31.3 | 99.2 |
| D | 0.0 | 0.0 | 5.4 | 69.0 |
| E | 41.3 | 62.2 | 13.7 | 45.7 |
| F | 21.3 | 65.6 | 44.3 | 69.0 |
| G | 22.2 | 23.8 | 59.4 | 24.4 |

Using Method VIII in place of Method VI, the dentin adhesion of modified adhesives B and E increased to 46.1 and 35.6 kg/cm$^2$, respectively. These products are sold as light-curable adhesives, and it is believed that they may contain photosensitive catalyst in their resin portions. No attempt was made to remove such catalyst. The effect of the catalyst upon the photoinitiator of the invention, and/or the absence of the ingredients contained in the liquid portion of products B and E, may have been responsible for the decrease in adhesion to dentin noted when these products were modified as shown above. The remaining commercially available dental adhesive products exhibited increased adhesion to dentin and glass when modified as shown above. In addition, each of these commercially available dental adhesive products (all of which are sold as two-part compositions) was reformulated in a more convenient one-part package when modified as shown above.

**EXAMPLE 8**

Two fluoride-containing dental adhesives were prepared as follows:

Formulation A

| Ingredient | Amount, % |
|---|---|
| Adduct of EXAMPLE 1 | 97.0 |
| Fumed silica | 2.0 |
| Sodium fluoride | 0.5 |
| MOST | 0.5 |

Formulation B

| Ingredient | Amount, % |
|---|---|
| Adduct of EXAMPLE 1 | 95.5 |
| Fumed silica | 2.0 |
| Zirconium fluoride | 2.0 |
| MOST | 0.5 |

The fumed silica used in each formulation was "Aerosil R-972" (DeGussa). The ingredients in each formulation were stirred together in a "Waring" blender until a homogeneous mixture was obtained. Formulation B was evaluated for adhesion to dentin using Method VI. It exhibited an average measured adhesive shear strength of 75.8 kg/cm$^2$.

Cured discs (one mm thick by 20 mm diameter) of each formulation were molded in a metal ring mold having polyester film on its upper and lower surfaces, cured for 60 seconds on each side, weighed and immersed in deionized water. By measuring the fluoride content of the water over time (using a millivoltmeter equipped with a fluoride-selective electrode), the extent to which fluoride had leached from each formulation could be measured. Set out below in TABLE VIII are the elapsed immersion time and cumulative amount of leached fluoride for each formulation.

**TABLE VIII**

| Elapsed immersion time | Cumulative leached fluoride, micrograms F per gram of adhesive: Formulation A | Formulation B |
|---|---|---|
| 4 Hours | 25 | 71 |
| 1 Day | 49 | 113 |
| 2 Days | 65 | 133 |
| 5 Days | 112 | 204 |
| 13 Days | 158 | 306 |
| 19 Days | 219 | 414 |
| 60 Days | 411 | 570 |

The above example illustrates dental adhesives containing leachable fluoride.

**EXAMPLE 9**

Adhesion to Cured Composite

Two commercially available light-curable restoratives ("Silux" restorative and "P-30" resin bonded ceramic, 3M) were formed into cured substrates by molding a 2-mm thick by 20 mm diameter disc of each restorative in a metal ring mold having polyester film on its upper and lower surfaces, curing the disc for 60 seconds on each side, and then further curing the disc in an illuminated vacuum chamber ("ESPE Visio Beta", commercially available from Premier Dental Products Company) for 2 minutes. The cured substrates were bonded to uncured restorative using the composition of Run 2 of EXAMPLE 5 and Method VI. Set out below in TABLE IX are the run number, substrate, restorative, and average measured adhesive shear strength for each run.

TABLE IX

| Run | Substrate | Restorative | Adhesion, kg/cm$^2$ |
|---|---|---|---|
| 1 | "Silux" | "Silux" | 79.9 |
| 2 | "Silux" | "P-30" | 62.0 |
| 3 | "P-30" | "Silux" | 138.6 |
| 4 | "P-30" | "P-30" | 145.6 |

In each run failure occurred within the substrate, indicating that the bond strength exceeded the cohesive (internal) strength of the substrate.

**EXAMPLE 10**

Adhesion to Porcelain

Several samples of dental porcelain ("Trubyte Bioform" porcelain anterior teeth, commercially available from the York Division of Dentsply International) were embedded in an acrylic disk using the method of EXAMPLE 1 and ground flat using a Grade 320 abrasive disc. The polished porcelain surface was cleaned using a 60-second application of 35% σ-phosphoric acid, rinsed with distilled water and dried with oil-free air. Using the composition of Run 2 of of EXAMPLE 5 and Method I, a layer of dental adhesive and a button of restorative were adhered to the porcelain, cured, and evaluated for shear strength. The average measured shear strength was 85.0 kg/cm$^2$, a value approaching the cohesive strength of dental porcelain.

**EXAMPLE 11**

Adhesion to Alloys

Using the method of EXAMPLE 10, but without using an acid cleaning step, the bond strength of the composition of Run 2 of EXAMPLE 5 was evaluated on several commercially available dental alloys. For each evaluation one to five samples were employed. Set out below in TABLE X are the product names for the alloys employed, the approximate composition (where known) of the major alloy ingredients, and the average measured adhesive shear strengths (shown under the heading "With photoinitiator") obtained using the composition of the invention. Also included within TABLE X are the average measured adhesive shear strengths (shown under the heading "With CPQ") obtained using the comparison two-part light curable dental adhesive of EXAMPLE 1.

TABLE X

| Metal | Composition | Adhesion, kg/cm$^2$ With photoinitiator | With CPQ |
|---|---|---|---|
| "Premium Talladium"(1) | 76:15:5:2 Ni:Cr:Mo:Be | 100.0 | 54.6 |
| "Super 12"(2) | -- | 76.1 | 72.2 |
| "Rexillium III"(3) | 74-78:12-15:4-6:≤1/8 Ni:Cr:Mo:Be | 80.1 | 46.0 |
| "Jel 5"(4) | 53.5:38.9 Pd:Ag | 59.0 | 37.0 |
| "Stroma"(5) | 84.8 Pd | 95.1 | 32.3 |
| "PTM-45"(4) | 45:40:5 Au:Pd:Ag | 75.1 | 31.0 |

Notes for entries in TABLE X:

(1) Talladium Inc.

(2) Dental Alloy Products, Inc.

(3) Jeneric Industries, Inc.

(4) Jelenko Dental Health Products.

(5) Unitek, Inc.

The above example shows that the compositions of the invention exhibit enhanced adhesion to a variety of alloys commonly used in dentistry.

Various modifications and alterations of this invention will become apparent to those skilled in the art without departing from the scope and spirit of this invention, and it should be understood that this invention is not limited to the illustrative embodiments set forth herein.

**Claims**

1. Light-curable dental adhesive composition, comprising phosphorus-containing free-radically polymerizable compound suitable for use in the oral environment, photoinitiator, and optional ketone, characterized in that said photoinitiator comprises chromophore-substituted halomethyl-s-triazine or chromophore-substituted halomethyl-oxadiazole.

2. A composition according to claim 1, characterized in that said phosphorus-containing compound contains one or more phosphorus atoms bonded (a) through a P-OR or P-R linkage where R is a carbon, nitrogen or sulfur atom, to a hydrocarbyl radical containing one or more olefinically unsaturated groups, and (b) to one or more (i) halogen atoms through a P-Cl, P-Br or P-F linkage, (ii) active hydrogen atoms through a P-H or P-OH linkage, or (iii) substituted or unsubstituted hydrocarbyl groups through a P-OR or P-R linkage.

3. A composition according to claim 1, characterized in that said phosphorus-containing compound comprises a halophosphorus ester of Bis-GMA.

4. A composition according to any preceding claim, characterized in that said triazine or oxadiazole is substituted with a trihalomethyl group.

5. A composition according to claim 4, characterized in that said trihalomethyl group comprises a trichloromethyl group.

6. A composition according to any preceding claim, characterized in that said chromophore comprises a substituted or unsubstituted styryl, aryl, or alkyl group.

7. A composition according to any preceding claim, characterized in that said chromophore comprises an alkyl-or alkoxy-substituted styryl group.

8. A composition according to any preceding claim, characterized in that said triazine has the formula:

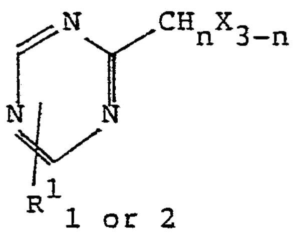

where n is zero to two, each X is independently chlorine or bromine, and each $R^1$ is independently a chromophoric moiety.

9. A composition according to any preceding claim, characterized in that said oxadiazole has the formula:

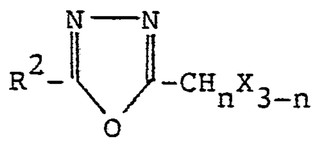

where n is zero to two, each X is independently chlorine or bromine, and $R^2$ is a chromophoric moiety.

10. A composition according to any preceding claim, characterized in that said triazine or oxadiazole is selected from the group consisting of 2-( p-methoxystyryl)-4,6-bis(trichloromethyl)-s-triazine, 2-(p-methylstyryl)-4,6-bis(trichloro-methyl)-s-triazine, 2-(2,4-dimethylstyryl)-4,6-bis(tri-chloromethyl)-s-triazine, 2-(mmethoxystyryl)-4,6-bis(tri-chloromethyl)-s-triazine, and 2-trichoromethyl-5-(3′,4′-dimethoxystyryl)-1,3,4-oxadiazole.

11. A composition according to any preceding claim, characterized by containing 0.05 to five percent by weight of said triazine or oxadiazole, based on the weight of said phosphorus-containing compound.

12. A composition according to any preceding claim, further characterized by comprising ketone.

13. A composition according to claim 12, characterized in that said ketone comprises diketone.

14. A composition according to claim 12, characterized in that said ketone comprises α-diketone.

15. A composition according to claim 12, characterized in that said ketone comprises camphorquinone, acenaphthenequinone, phenanthrenequinone or 1,4-naphthaquinone.

16. A composition according to claim 12, characterized by containing 0.05 to five weight percent of said triazine or oxadiazole, based on the weight of said phosphorus compound, and 0.05 to five weight percent of said ketone, based on the weight of said composition.

17. A composition according to claim 12, further characterized by comprising fluoride.